# EUROPEAN PATENT APPLICATION

(11) **EP 4 841 394 A1**
(43) Date of publication of application: **18.11.2026**
(21) Application number: 26177848.4
(22) Date of filing: 11.05.2026
(51) Int. Cl.: G06F 1/3287, A61B 5/0205, G06F 1/324, G06F 1/3203, G06F 1/3293

(54) **BATTERY POWERED MEDICAL DEVICE WITH POWER OPTIMIZATION**

(30) Priority: 12.05.2025 US 202563803978 P; 04.05.2026 US 202619666403
(71) Applicant: Welch Allyn, Inc., Skaneateles Falls, NY 13153-0220 (US)
(72) Inventor: FALLAT, David M., Batesville, 47006 (US); BABSON, David M., Batesville, 47006 (US); COON, Kenneth V., Batesville, 47006 (US); BRIGGS, Thomas Henry, Batesville, 47006 (US); MCMAHON, Michael T., Batesville, 47006 (US); KNIERIEM, Alan S., Batesville, 47006 (US)
(74) Representative: Reddie & Grose LLP

(57) **Abstract**

A battery powered medical device (20) having a housing (22), a battery (80) configured to supply electrical power, an electronic display (24), a plurality of monitoring devices (28, 32, 34, 36) operatively coupled to the battery (80) to receive electrical power from the battery (80), and a plurality of processing units (92). The plurality of processing units (92) controls the electrical power supplied to each of the electronic display (24), the plurality of monitoring devices (28, 32, 34, 36) and the plurality of processing units (92) and enters a sleep mode which turns OFF one or more of the plurality of processing units (92), backlighting of the electronic display (24) and the plurality of monitoring devices (28, 32, 34, 36). The first processing unit (52) remains turned ON and with a reduced processing speed in the sleep mode, and when a use mode is requested, the first processing unit (52) turns ON one or more of the plurality of processing units (92), the backlighting of the electronic display (24) and the plurality of monitoring devices (28, 32, 34, 36).

## Description

The present disclosure generally relates to battery powered electronic devices such as a medical monitor device, and more particularly relates to a medical device configured to operate with optimized battery power consumption.

Electronic medical devices commonly exist which employ high capacity processors that drive electric loads including large liquid crystal displays and patient monitoring devices that are configured to measure medical conditions such as pulse oximetry, non-invasive blood pressure, temperature, end-tidal CO2 (ETCO2) and respiration, for example. The medical devices are often also configured with electric power delivery outlets may communicate with and power electric loads. The various accessories associated with the electronic medical device are typically battery powered over extensive periods of time and require a significant amount of battery supplied energy. The medical devices are often configured to be transportable and typically must be readily available for use while battery powered and therefore need to deliver battery power in a timely manner. It would be desirable to provide for an electronic medical device that optimizes the battery power consumption, is readily available for use, and minimizes battery size and device weight.

### SUMMARY OF THE DISCLOSURE

According to one aspect of the present disclosure, a battery powered medical device has a housing, a battery configured to supply electrical power, an electronic display, a plurality of monitoring devices operatively coupled to the battery to receive electrical power from the battery, and a plurality of processing units including at least a first processing unit and a second processing unit. The plurality of processing units controls the electrical power supplied to each of the electronic display, the plurality of monitoring devices and the plurality of processing units and are configured to enter a sleep mode which turns OFF one or more of the plurality of processing units, backlighting of the electronic display and the plurality of monitoring devices. The first processing unit remains turned ON in the sleep mode, and when a use mode is requested, the first processing unit turns ON the one or more of the plurality of processing units, the backlighting of the electronic display and the plurality of monitoring devices.

According to another aspect of the present disclosure, a battery powered medical monitor device has a housing, a battery configured to supply electrical power, an electronic display, one or more power ports configured to supply the electric power from the battery to power a plurality of electrical loads, a plurality of monitoring devices operatively coupled to the one or more power ports to receive electrical power from the battery, and a plurality of processing units including at least a first processing unit and a second processing unit. The plurality of processing units controls the electrical power supplied to each of the electronic display, the plurality of monitoring devices and the plurality of processing units, and are configured to enter a sleep mode which turns OFF one or more of the plurality of processing units, backlighting of the electronic display and the plurality of monitoring devices. The first processing unit remains turned ON in the sleep mode and a processing speed of the first processing unit is reduced in the sleep mode compared to the use mode. When a use mode is requested, the first processing unit turns ON the plurality of processing units, the backlighting of the electronic display and the plurality of monitoring devices.

According to another aspect of the present disclosure, a battery powered electronic device has a housing, a battery configured to supply electrical power, an electronic display, a plurality of power ports configured to power a plurality of electrical loads, and a plurality of processing units including at least a first processing unit and a second processing unit. The plurality of processing units controls the electrical power supplied to each of the electronic display, the plurality of power ports and the plurality of electrical loads, and are configured to enter a sleep mode state which turns OFF one or more of the plurality of processing units, backlighting of the electronic display and the plurality of power ports. The first processing unit remains turned ON in the sleep mode state and a processing speed of the first processing unit is reduced in the sleep mode compared to the active use mode state. When an active use mode is requested, the first processing unit turns ON the plurality of processing units, the backlighting of the electronic display and the plurality of electrical loads.

According to another aspect of the present disclosure, a method of operating a battery powered medical device having a battery, an electronic display, a plurality of monitoring devices and a plurality of processing units is included. The method comprises the steps of charging the battery, turning ON the electronic display, the plurality of monitoring devices and the plurality of processing units in an active use mode, detecting non-use of the device during a first time period or a user request for sleep mode and entering the sleep mode, turning OFF one or more of the plurality of processing units and maintaining at least a first processing unit turned ON in the sleep mode, turning OFF backlighting of the electronic display in the sleep mode, turning OFF the plurality of monitoring devices in the sleep mode, detecting a request for the active use mode of the medical device while in the sleep mode, and turning ON all of the plurality of processing units, the backlighting of the electronic display and the plurality of monitoring devices in the active use mode.

The invention will now be further described by way of example with reference to the accompanying drawings, in which:
FIG. 1 is a front perspective view of an electronic device in the form of a medical monitor device supported on a transportable stand in a medical facility, according to one example;
FIG. 2 is an enlarged front perspective view of the medical monitor device shown in FIG. 1 that may be supported on the stand or a wall mount and transported as a portable device;
FIG. 3 is a bottom view of the medical monitor device shown in FIG. 1 illustrating the power ports for connecting with electrical loads;
FIG. 4 is a block diagram illustrating the medical monitor device connected to a plurality of electrical powered loads which are controlled pursuant to a battery power optimization routine; and
FIG. 5 is a flow diagram illustrating the battery power optimization routine executed by the computer engine in the medical monitor device.

The present illustrated embodiments reside primarily in combinations of method steps, systems, devices, and apparatus components related to systems, methods, and programs for optimizing the battery power consumption associated with an electronic medical device. Accordingly, the apparatus components and method steps have been represented, where appropriate, by conventional symbols in the drawings, showing only those specific details that are pertinent to understanding the embodiments of the present disclosure so as not to obscure the disclosure with details that will be readily apparent to those of ordinary skill in the art having the benefit of the description herein. Further, like numerals in the description and drawings represent like elements.

The specific structures and processes illustrated in the attached drawings, and described in the following specification are simply exemplary embodiments of the inventive concepts defined in the appended claims. Hence, specific dimensions and other physical characteristics relating to the embodiments disclosed herein are not to be considered as limiting, unless the claims expressly state otherwise.

The terms "including," "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. An element preceded by "comprises a . . . " does not, without more constraints, preclude the existence of additional identical elements in the process, method, article, or apparatus that comprises the element.

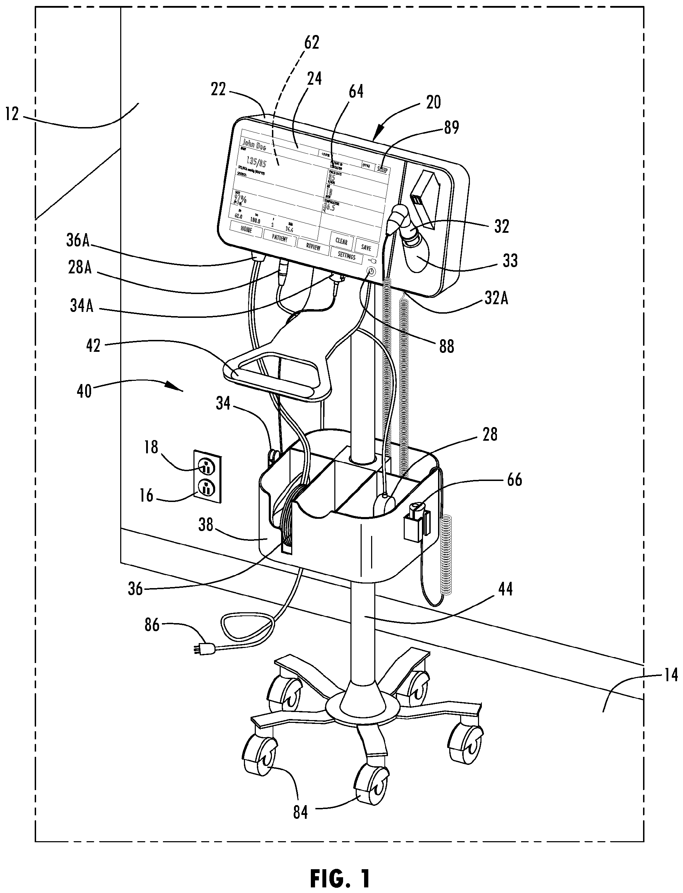

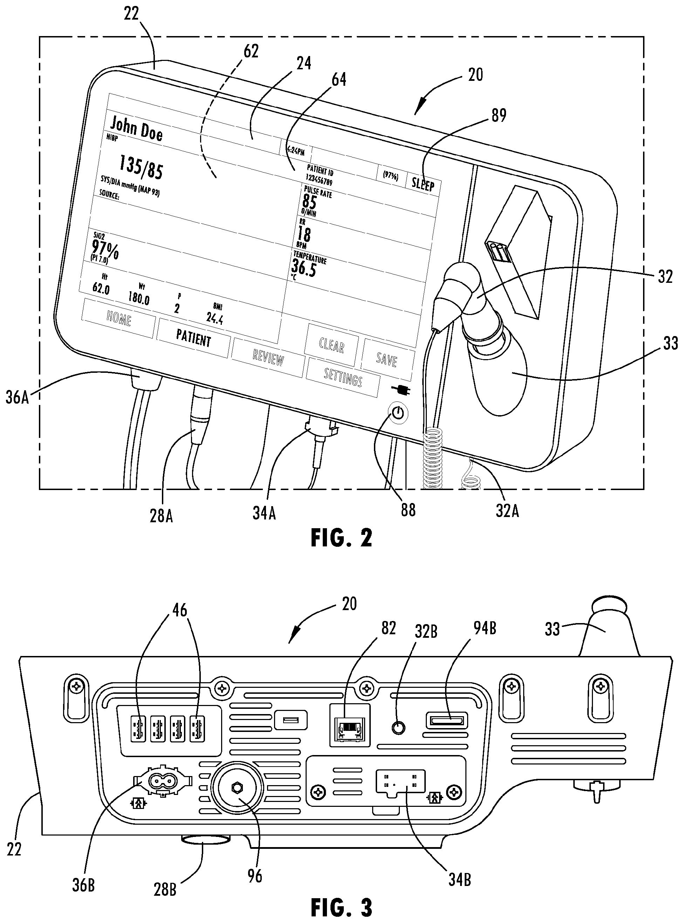

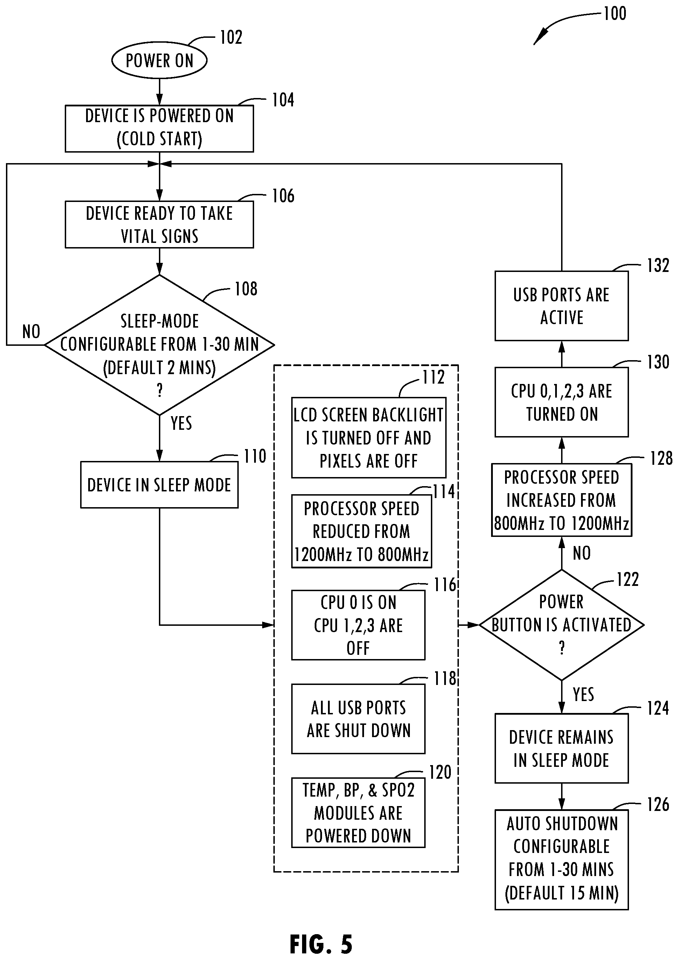

Referring to FIGS. 1-5, an electrical battery powered medical device 20 configured with power optimization is generally illustrated according to one embodiment. The medical device 20 has a housing 22, a battery 80 configured to supply electrical power, an electronic display 24 and a plurality of patient monitoring devices operatively coupled to the battery 80 to receive electrical power from the battery 80. The medical device 20 also has a plurality of processing units 92 including at least a first processing unit 52 and a second processing unit 56. The plurality of processing units 92 controls the power supplied to each of the electronic display, the plurality of monitoring devices and the plurality of processing units. The plurality of processing units are configured to enter a sleep mode upon detecting an inactivity trigger setpoint to reduce electrical power consumption and hence conserve the electrical energy stored in the battery 80. The sleep mode turns OFF at least some of the plurality of processing units 92, backlighting of the electronic display 24 and the plurality of monitoring devices. The processing units 92 further maintain the first processing unit 52 turned ON in the sleep mode, and when a use mode is requested, the first processing unit 52 turns ON the plurality of processing units 92, the backlighting of the LCD display 24 and the plurality of monitoring devices.

Referring to FIG. 1, an electronic medical device 20 is generally illustrated configured as a medical monitor device generally shown located within a medical facility 10. The medical monitor device 20 is shown and described herein according to one example as a portable medical monitor device 20 that may be supported on a transportable stand 40 that makes the medical monitor device 20 readily available to a medical practitioner user such as a doctor, nurse or physician's assistant, for example. The medical monitor device 20 may otherwise be supported on a wall mount stand or other supporting structure and may be hand held and easily transported by a user. The medical monitor device 20 is configured to be battery powered by a battery to allow the medical monitor device 20 to be readily transported on the stand or by hand held transportation by a user and used remotely at one or more locations without requiring connection to a fixed AC power supply. The battery provides electric energy to power the device 20 when a fixed AC power source is not connected to the medical monitor device 20 and may be electrically recharged when connected to the AC power source when additional battery energy is needed. The medical facility 10 shown in this example may be any suitable location for providing treatment for a patient with the medical monitor device 20. It is contemplated that other types of electronic medical devices such as medical beds, examination tables, operating tables and other electrically powered devices that may employ battery power for supplying electricity to electrical loads may be contemplated, without departing from the teachings herein.

The medical monitor device 20 is a battery operated portable electronic device that may be transported by a user to different locations such as throughout different locations within a medical facility 10 for use in monitoring and diagnosing patients, such as medical patients in a hospital. The medical monitor device 20 generally has a housing 22 and an electronic display 24 flush mounted on the front side of the housing 22 that serves as a Human Machine Interface (HMI) to output visual content on the display screen and may also operate as an input device such as via a touch screen input. The electronic display 24 may be a liquid crystal display (LCD), for example. The electronic display 24 generally includes illuminated backlighting to allow viewers to more clearly see the display, particularly in low light conditions. The display backlighting is powered via electricity and draws electric current and therefore is an electrical load. Backlighting of the electronic display 24 may consume a substantial amount of electrical power. The electronic display 24 may also include pixels that draw electric current and are therefore electrical loads.

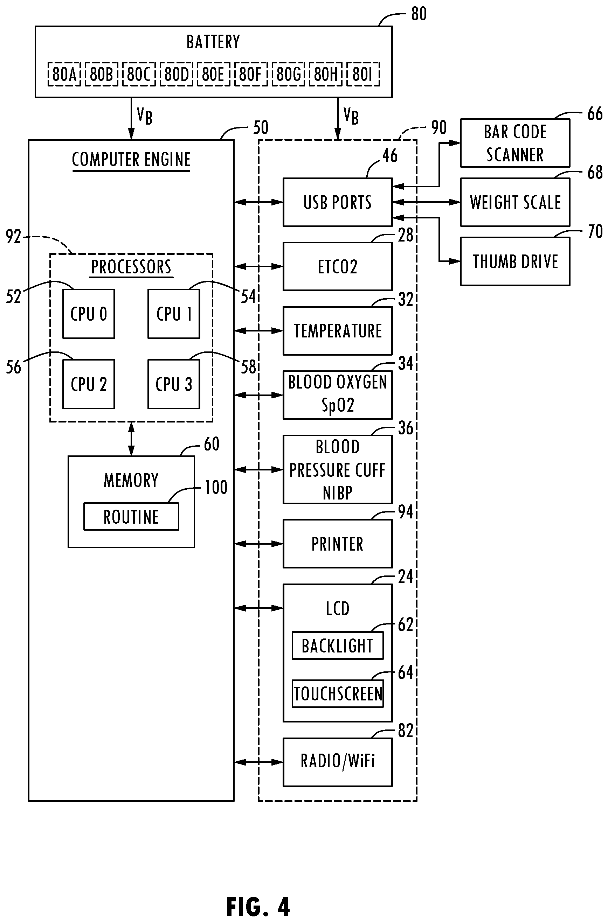

The medical monitor device 20 has a battery 80 shown in FIG. 4 which may be assembled within a battery compartment in the housing 22 which includes one or more battery cells 80A-80I for providing electrical power to power the various electrical loads. The battery 80 is a rechargeable battery that may be recharged to replenish the available battery energy stored in the one or more battery cells. The battery 80 is configured to store a limited amount of energy sufficient to power the electrical loads for a substantial time of use and allow for a compact and lightweight portable device. The medical monitor device 20 is controlled such that the battery 80 supplies electrical power to the various electrical loads and is readily available for use for a substantial period of time such as 10 hours or more, for example. The medical monitor device 20 has a plurality of electrical loads as shown in FIGS. 1-3 that may be plugged into the medical monitor device 20 that draw electrical power from the battery 80. In the example shown, the plug-in electrical loads may include a carbon monoxide monitor 28 having a connector 28A connected at power port 28B on the bottom of the medical monitor device 20 for measuring concentration of carbon monoxide in the blood of a patient at the end of a breath, also referred to as ETCO2. The plug-in electrical loads may also include a blood oxygen sensor monitor 34 having a connector 34A connected at power port 34B, also referred to as a saturation of peripheral oxygen sensor monitor (SPO2). The plug-in electrical loads further include a non-invasive blood pressure monitor 36 (NIBP) having a terminal connector 36A connected at power port 36B, and a temperature sensor monitor 32 having a terminal connector 32A connected at power port 32B. The temperature sensor monitor 32 may be stored in a holding tray 33 on the front of the medical monitor device 20. Additional loads such as a printer 94 and other electrical powered devices may also be connected to and powered by the medical monitor device 20. The medical monitor device 20 also includes a plurality of universal serial bus (USB) ports 46 which are configured to supply electrical power to and communicate with a plurality of electronic devices. For example, the USB ports 46 may connect to and power a bar code scanner 66, a weight scale 68 and a thumb drive 70, for example. In the example shown, four USB ports are provided. However, it should be appreciated that any of a number of USB ports, including USBA and USBC ports, may be included to connect various electric powered electrical loads to the medical monitor device 20 such that the electrical loads are powered by the battery 80.

The medical monitor device 20 is shown in FIG. 1 supported on a mobile roller stand 40 which, in one example, includes an upright elongated support structure 44 supported by wheels 84 on the bottom side to provide rolling mobility to the stand 40. The stand 40 also includes a tray 38 which may be configured to contain and hold the various accessories such as the monitoring devices connected as electrical loads to the medical monitor device 20. The stand 40 further includes a handle 42. It should be appreciated that a user, such as a medical practitioner, may transport the medical monitor device 20 by maneuvering the handle 42 and rolling the wheels 84 to move the stand 40 from one location to another location and use the medical monitor device 20 while powered by the battery 80 and thus does not require any electrical power cords to be connected to the AC main power supply 16 as long as the battery 80 has a sufficient energy charge. It should further be appreciated that the medical monitor device 20 may be stowed on a wall mount stand or placed elsewhere. With either a wall mount stand or the roller stand 40, the medical practitioner may further remove the medical monitor device 20 from the stand and transport the medical monitor device 20 as a hand held device to other locations such as to other medical examination rooms, laboratories, waiting rooms, and any other location where the medical monitor device 20 may be efficiently powered by the battery 80 and readily used.

The medical facility 10 is shown in FIG. 1 equipped with a main alternating current (AC) power supply 16 such as power outlets on a wall or elsewhere in the medical facility 10. Accordingly, the main AC electrical power supplied by a power distribution network in the medical facility 10 may be distributed and used to recharge the battery 80 in the medical monitor device 20. The AC main power may be converted to direct current (DC) power via an AC to DC converter which may be connected to an AC electrical plug 86 to supply a DC voltage such as about 12.6 volts, for example. The battery 80 is a rechargeable battery pack having one or more rechargeable battery cells that provide electric energy to power the electrical loads, when the AC electrical plug 86 is disconnected from the AC main power supply 16, such as when the medical monitor device 20 is awaiting use or is used by a physician or other medical practitioner or if the AC main power supply 16 is not available.

The battery 80 may be configured to include any number of battery cells to achieve the desired voltage and energy supply. The battery 80 may be configured to include nine (9) lithium ion battery cells 80A-80I as shown in FIG. 4, according to one example. The plurality of battery cells 80A-80I may be electrically connected in a series and parallel in a battery compartment housing. Each lithium ion battery cell is rechargeable and may have a nominal voltage of about 3.6 volts and may be charged to a voltage of about 4.2 volts such that the battery 80 with three groups of three series connected battery cells connected in parallel has a battery voltage output VB with a total nominal voltage of about 10.8 volts and a full charging voltage of about 12.6 volts, according to one example. A battery charger may be connected to the electrical plug 86 or provided in the medical monitor device 20 that is configured to charge the rechargeable battery 80 by supplying electrical power from the AC power supply 16 received via the electrical plug 86 which is configured to engage an outlet 18 and which is converted from AC electrical power to DC electrical power and used to charge the rechargeable battery 80.

The medical monitor device 20 is configured to include various electrically powered devices that may be activated and electrically powered by the battery 80 to thereby define electrical loads. The medical monitor device 20 is illustrated in FIG. 4 having a computer engine 50 and various electrical load devices 90 including the USB ports 46 and any electrically powered devices connected to the USB ports 46 such as a bar code scanner 66, a weight scale 68 and a thumb drive 70, for example. Other electrical load devices 90 connected to the power ports include the ETCO2 monitor 28, the temperature monitor 32, the blood oxygen monitor 34, the blood pressure monitor 26, a printer 94, the display 24 including the corresponding backlight 62 and touchscreen 64, and a radio/Wi-Fi wireless communication connectivity device 82. The radio/Wi-Fi wireless communication connectivity device includes communication circuitry that may remain activated in the sleep mode in one embodiment to maintain wireless communication and prevent delayed rebooting of the medical monitor device 20 from the sleep mode to the use mode. Each of the remaining electrical load devices 90 are connected to receive electrical power from the battery 80 and may be controlled by the computer engine 50 to turn ON or activate and to turn OFF or deactivate the corresponding electrical load device 90 pursuant to a battery optimization control routine 100 which is stored in memory 60 of the computer engine 50. Each of the electrical load devices and the computer engine 50 are configured to receive electrical power from the battery 80 so as to power the computer engine 50 and the various electrical load devices that are turned ON or activated. The battery optimization routine 100 advantageously controls the various electrical load devices to optimize the battery power consumption so as to extend the charge life of the battery 80 and to provide for a fast start-up power ON of the computer engine 50 and hence the medical monitor device 20.

The computer engine 50 also referred to as the processor and the controller, includes a plurality of processors 92 referred to as processing units or cores that can execute instructions simultaneously. In one example, the computer engine 50 is shown configured as a multi-core processor having four processors in the form of central processing units (CPUs). The four central processing units include a first central processing unit (CPU0) 52, a second central processing unit (CPU1) 54, a third central processing unit (CPU2) 56 and a fourth central processing unit (CPU3) 58. Each of the central processing units 52, 54, 56 and 58 are digital processing units that includes digital circuitry that may be fabricated on a semiconductor computer chip and may fetch, decode and execute programmed instructions stored in memory 60 to process data and one or more routines and may generate outputs. The four processing units may be configured with four corresponding cores manufactured on a common semiconductor chip.

The four processing units 52-58 advantageously provide for a selectable amount of processing power and consume electrical power from the battery 80 as electrical loads such that the amount of battery power consumed may vary by controlling which processing units are turned ON and OFF and the processing speed of the processing units that are turned ON. For example, when the medical monitor device 20 is not utilized such that there is no user input for a first continuous time period such as two minutes, the medical monitor device 20 may enter a sleep mode state with reduced processing capability by deactivating one or more of the processing units and with the processing speed of one or more processing unit reduced so as to consume less electrical power from the battery 80. The sleep mode state may also be entered by a user activating a sleep mode button icon 98 on the medical monitor device 20, such as touching the icon presented within a menu on the touchscreen display. In addition, the medical monitor device 20 may turn OFF certain electrical loads to conserve electrical power in the sleep mode state. The medical monitor device 20 advantageously maintains at least one of the processing units turned ON and at a reduced processing speed so as to allow for fast wakeup processing of the computer engine 50 and the medical monitor device 20 from the sleep mode state when a user activates the power ON input button 88 to turn the medical monitor device 20 back ON at full capacity in a use mode state. By shutting OFF the certain electrical loads, turning OFF at least some of the processing units, and slowing the speed down of the one or more processing units that are turned ON, reduced power consumption and extended battery life for a given charge can be realized with the medical monitor device 20.

The power optimization routine 100 is stored in memory 60 and executed by the processors 92. The power optimization routine 100 is illustrated in FIG. 4, according to one example. The power optimization routine 100 begins at step 102 with the medical monitor device 20 being powered ON when a user activates the power ON input button. Routine 100 then proceeds to step 104 where the medical monitor device is powered ON from the fully inactive state with a cold start, with the cold start starting with the inactive state where all of the central processing units are turned OFF and no electric power is provided to the electrical loads. It should be appreciated that the time required to power ON the device from the inactive state to the use state with a cold start is significant and, for example, can, in one example, take about 28 seconds for the processors to reboot, and the radio/Wi-Fi connectivity can take about two minutes, for example, to achieve connectivity. Once the medical monitor device is powered ON during the cold start, all of the processing units are activated and all of the electrical loads are activated and powered with electrical power. The power optimization routine 100 then proceeds to step 106 wherein the medical monitor device powered up and is ready to perform vital signs monitoring of a patient. The power optimization routine 100 then proceeds to step 108 to determine if the medical monitor device is not active and has entered the sleep mode state due to non-use of the medical monitor device for the first time period or receives a user request to enter the sleep mode. The sleep mode may be entered after the first time period of continuous non-use, such as two minutes, according to one example. It should be appreciated that a typical sleep mode state may be activated for a first time period of non-use of 1-30 minutes, for example. If the medical monitor device 20 is not operating in the sleep mode state, power optimization routine 100 returns to step 106 where the medical monitor device remains powered and ready to monitor a patient such as for vital signs.

Once the medical monitor device 20 enters the sleep mode state due to continuous non-use for a minimum of a first time period or a user request, the power optimizer routine 100 proceeds to step 110 wherein the medical monitor device enters the sleep mode. Once in the sleep mode state, power optimization routine 100 performs the following steps: the LCD screen backlight for the display is turned OFF and the pixels are OFF at step 112, the processor speed for one or more of the processing units that remain ON is reduced from a full first processing speed such as 1,200 MHz to a reduced second processing speed such as 800 MHz at step 114, CPU0 remains ON, while CPUs 1, 2 and 3 are turned OFF at step 116, all of the USB ports are shown down at step 118, and the temperature monitor, blood pressure monitor and oxygen monitor modules are powered down at step 120. It should be appreciated that at this point only one of the CPUs, CPU0, remains powered and operational and is operating at a reduced processing speed to conserve electrical energy supplied by the battery 80. All of the other processors and electrical loads with the exception of the radio/Wi-Fi connectivity 82 are shut down at the same time. The medical monitor device remains in this sleep mode state to conserve power until a user activates the power ON input button on the touch screen display which, at decision step 122, is detected. If the power ON input button has not been activated, the medical monitor device remains in the sleep mode state at step 124, and eventually may enter an automatic shutdown state which is the fully inactive state to turn OFF all of the electric loads including all of the processor units at step 126. The automatic shutdown state at step 126 may occur after a second time period of continuous non-use such as 1-30 minutes, according to one example. According to a specific example, the automatic shutdown state may occur after the second time period of continuous non-use of about 50 minutes.

If the power ON input button is activated by a user at decision step 122, power optimization routine 100 proceeds to step 128 to increase the processing speed of the one or more processing units that are ON from the reduced speed of 800 MHz to the normal operating speed of 1200 MHz, for example. It should be appreciated that other processing speeds and reductions in the processing speed may be used to conserve energy. Next, routine 100 turns ON all of the processing units (CPUs) that were turned OFF, including CPU1, CPU2 and CPU3 at step 130. Next, routine 100 turns ON power to the USB ports at step 132 before returning to step 106. It should be appreciated that the amount of time required to activate the power OFF processing units and other loads to the use mode state is fast and minimal such as, for example, about 100 milliseconds. In order to further assume a fast return of full functionality of the device, the radio/Wi-Fi connectivity in one embodiment may be kept activated during the sleep mode state.

The medical monitor device 20 advantageously includes the power optimization routine 100 which controls the various electric loads to turn OFF certain devices in the sleep mode state to conserve battery power in addition to turning OFF one or more processing units and reducing the processing speed of one or more processing units, thereby conserving battery energy and resulting in an efficient medical monitor device 20 with an extended usage time and enhanced speed activation from the sleep mode state. It should be appreciated that the disclosure is described herein in terms of a medical device, however, the teachings of the present disclosure may be broader and may be applicable to any battery operated electronic device that benefits from efficiently reducing power consumption to save battery power.

Embodiments of the invention can be described with reference to the following numbered clauses, with preferred features laid out in the dependent clauses:
1. A battery powered medical device comprising:
   a housing;
   a battery configured to supply electrical power;
   an electronic display;
   a plurality of monitoring devices operatively coupled to the battery to receive electrical power from the battery; and
   a plurality of processing units including at least a first processing unit and a second processing unit, wherein the plurality of processing units controls the electrical power supplied to each of the electronic display, the plurality of monitoring devices and the plurality of processing units, wherein the plurality of processing units are configured to enter a sleep mode which turns OFF one or more of the plurality of processing units, backlighting of the electronic display and the plurality of monitoring devices, and wherein the first processing unit remains turned ON in the sleep mode, and when a use mode is requested, the first processing unit turns ON the one or more of the plurality of processing units, the backlighting of the electronic display and the plurality of monitoring devices.
2. The medical device of clause 1, wherein a processing speed of the first processing unit is reduced in the sleep mode compared to the use mode.
3. The medical device of either clause 1 or clause 2, further comprising one or more power ports configured to power one or more electrical loads.
4. The medical device of clause 3, wherein the plurality of processor units further turns OFF the power supplied to the one or more power ports in the sleep mode, and reactivates the one or more power ports when the use mode is requested.
5. The medical device of any preceding clause, wherein the plurality of processor units includes the first processing unit, the second processing unit, a third processing unit and a fourth processing unit, and wherein the second, third and fourth processing units are turned OFF in the sleep mode.
6. The medical device of any preceding clause, wherein the medical device is a medical monitor device having the plurality of monitoring devices configured to monitor medical conditions of a patient.
7. The medical device of clause 6, wherein the plurality of monitoring devices comprises one or more of a temperature sensor monitor, a blood pressure sensor monitor, an oxygen sensor monitor and a carbon dioxide sensor monitor.
8. The medical device of any preceding clause, wherein the plurality of monitor devices comprises a temperature sensor monitor, a blood pressure sensor monitor and an oxygen sensor monitor.
9. The medical device of any preceding clause, further comprising communication circuitry for wireless communication, wherein the communication circuitry remains ON in the sleep mode.
10. The medical device of any preceding clause, wherein the battery comprises a plurality of battery cells.
11. The medical device of clause 10, wherein the plurality of battery cells comprises a plurality of lithium ion battery cells.
12. A battery powered medical monitor device comprising:
   a housing;
   a battery configured to supply electrical power;
   an electronic display;
   one or more power ports configured to supply the electric power from the battery pack to power one or more electrical loads;
   a plurality of monitors operatively coupled to the one or more power ports to receive electrical power from the battery pack; and
   a plurality of processing units including at least a first processing unit and a second processing unit, wherein the plurality of processing units controls the electrical power supplied to each of the electronic display, the plurality of monitoring devices and the plurality of processing units, wherein the plurality of processing units are configured to enter a sleep mode which turns OFF one or more of the plurality of processing units, backlighting of the electronic display and the plurality of sensors, and wherein the first processing unit remains turned ON in the sleep mode and a processing speed of the first processing unit is reduced in the sleep mode compared to the use mode, and when a use mode is requested, the first processing unit turns ON the plurality of processing units, the backlighting of the electronic display and the plurality of sensors.
13. The medical monitor device of clause 12, wherein the plurality of processor units turns OFF the one or more power ports in the sleep mode, and reactivates the one or more power ports when the use mode is requested.
14. The medical monitor device of either clause 12 or clause 13, wherein the plurality of processor units includes the first processing unit, the second processing unit, a third processing unit and a fourth processing unit, and wherein the second, third and fourth processing units are turned OFF in the sleep mode.
15. The medical monitor device of any one of clauses 12 to 14, wherein the plurality of monitors comprises one or more of a temperature sensor monitor, a blood pressure monitor, an oxygen sensor monitor and a carbon dioxide sensor monitor.
16. The medical monitor device of any one of clauses 12 to 14, wherein the medical monitor comprises a temperature sensor monitor, a blood pressure sensor monitor and an oxygen sensor monitor.
17. The medical monitor device of any one of clauses 12 to 16, further comprising communication circuitry for wireless communication, wherein the communication circuitry remains ON in the sleep mode.
18. The medical monitor device of any one of clauses 12 to 17, wherein the battery comprises a plurality of battery cells.
19. The medical monitor device of clause 18, wherein the plurality of battery cells comprises a plurality of lithium ion battery cells.
20. A battery powered electronic device comprising:
   a housing;
   a battery configured to supply electrical power;
   an electronic display;
   a plurality of power ports configured to power one or more electrical loads; and
   a plurality of processing units including at least a first processing unit and a second processing unit, wherein the plurality of processing units controls the electrical power supplied to each of the electronic display, the plurality of power ports and the plurality of loads, wherein the plurality of processing units are configured to enter a sleep mode state which turns OFF one or more of the plurality of processing units, backlighting of the electronic display and the plurality of power ports, and wherein the first processing unit remains turned ON in the sleep mode state and a processing speed of the first processing unit is reduced in the sleep mode compared to the active use mode state, and when an active use mode is requested, the first processing unit turns ON the plurality of processing units, the backlighting of the electronic display and the plurality of sensors.
21. The device of clause 20, wherein a plurality of sensors operatively coupled to the plurality of power ports to receive electrical power from the battery.
22. The device of either clause 20 or clause 21, wherein the plurality of processor units turns OFF the one or more power ports in the sleep mode, and reactivates the one or more power ports when the use mode is requested.
23. The device of any one of clauses 20 to 22, wherein the plurality of processor units includes the first processing unit, the second processing unit, a third processing unit and a fourth processing unit, and wherein the second, third and fourth processing units are turned OFF in the sleep mode.
24. The device of any one of clauses 20 to 23, wherein the medical device is a medical monitoring device having a plurality of inputs monitoring medical conditions.
25. The device of clause 24, wherein the plurality of monitoring devices comprises one or more of a temperature sensor, a blood pressure sensor, an oxygen sensor monitor and a carbon dioxide sensor.
26. The device of clause 24, wherein the medical monitor comprises a temperature sensor, a blood pressure sensor and an oxygen sensor monitor.
27. The device of any one of clauses 20 to 26, wherein the plurality of loads further comprises communication circuitry for wireless communication, wherein the communication circuitry remains ON in the sleep mode.
28. The device of any one of clauses 20 to 27, wherein the battery pack comprises a plurality of battery cells.
29. The device of clause 28, wherein the plurality of battery cells comprises a plurality of lithium ion battery cells.
30. A method of operating a battery powered medical device having a battery, an electronic display, a plurality of monitoring devices and a plurality of processing units, the method comprising the steps of:
   charging the battery;
   turning ON the electronic display, the plurality of monitoring devices and the plurality of processing units in an active use mode;
   detecting non-use of the device during a first time period or a user request for sleep mode and entering the sleep mode;
   turning OFF one or more of the plurality of processing units and maintaining at least a first processing unit turned ON in the sleep mode;
   turning OFF backlighting of the electronic display in the sleep mode;
   turning OFF the plurality of monitoring devices in the sleep mode;
   detecting a request for the active use mode of the medical device while in the sleep mode; and turning ON all of the plurality of processing units, the backlighting of the electronic display and the plurality of monitoring devices in the active use mode.
31. The method of clause 30, further comprising the step of reducing a processing speed of the first processing unit in the sleep mode compared to the active use mode.
32. The method of either clause 30 or clause 31, further comprising the step of turning OFF one or more power ports in the sleep mode and reactivating the one or more power ports in the active use mode.
33. The method of any one of clauses 30 to 32, wherein the step of turning OFF the one or more of the plurality of processing units comprises turning OFF a second processing unit, a third processing unit, and a fourth processing unit in the sleep mode.
34. The method of any one of clauses 30 to 33, wherein the medical device comprises a medical monitoring device having a plurality of inputs configured for monitoring medical conditions.
35. The method of any one of clauses 30 to 34, wherein the plurality of monitoring devices comprises one or more of a temperature sensor monitor, a blood pressure sensor monitor, an oxygen sensor monitor and a carbon dioxide sensor monitor.
36. The method of any one of clauses 30 to 34, wherein the medical monitor comprises a temperature sensor monitor, a blood pressure sensor monitor and an oxygen sensor monitor.

It will be understood by one having ordinary skill in the art that construction of the described disclosure and other components is not limited to any specific material. Other exemplary embodiments of the disclosure disclosed herein may be formed from a wide variety of materials, unless described otherwise herein.

For purposes of this disclosure, the term "coupled" (in all of its forms, couple, coupling, coupled, etc.) generally means the joining of two components (electrical or mechanical) directly or indirectly to one another. Such joining may be stationary in nature or movable in nature. Such joining may be achieved with the two components (electrical or mechanical) and any additional intermediate members being integrally formed as a single unitary body with one another or with the two components. Such joining may be permanent in nature or may be removable or releasable in nature unless otherwise stated.

As used herein, the term "about" means that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art. When the term "about" is used in describing a value or an end-point of a range, the disclosure should be understood to include the specific value or end-point referred to. Whether or not a numerical value or end-point of a range in the specification recites "about," the numerical value or end-point of a range is intended to include two embodiments: one modified by "about," and one not modified by "about." It will be further understood that the end-points of each of the ranges are significant both in relation to the other end-point, and independently of the other end-point.

The terms "substantial," "substantially," and variations thereof as used herein are intended to note that a described feature is equal or approximately equal to a value or description. For example, a "substantially planar" surface is intended to denote a surface that is planar or approximately planar. Moreover, "substantially" is intended to denote that two values are equal or approximately equal. In some embodiments, "substantially" may denote values within about 10% of each other, such as within about 5% of each other, or within about 2% of each other.

It is also important to note that the construction and arrangement of the elements of the disclosure, as shown in the exemplary embodiments, is illustrative only. Although only a few embodiments of the present innovations have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recited. For example, elements shown as integrally formed may be constructed of multiple parts, or elements shown as multiple parts may be integrally formed, the operation of the interfaces may be reversed or otherwise varied, the length or width of the structures and/or members or connector or other elements of the system may be varied, the nature or number of adjustment positions provided between the elements may be varied. It should be noted that the elements and/or assemblies of the system may be constructed from any of a wide variety of materials that provide sufficient strength or durability, in any of a wide variety of colors, textures, and combinations. Accordingly, all such modifications are intended to be included within the scope of the present innovations. Other substitutions, modifications, changes, and omissions may be made in the design, operating conditions, and arrangement of the desired and other exemplary embodiments without departing from the spirit of the present innovations.

It will be understood that any described processes or steps within described processes may be combined with other disclosed processes or steps to form structures within the scope of the present disclosure. The exemplary structures and processes disclosed herein are for illustrative purposes and are not to be construed as limiting.

## Claims

1. A battery powered medical device (20) comprising:
a housing (22);
a battery (80) configured to supply electrical power;
an electronic display (24);
a plurality of monitoring devices (28, 32, 34, 36) operatively coupled to the battery (80) to receive electrical power from the battery (80); and
a plurality of processing units (92) including at least a first processing unit (52) and a second processing unit (54), wherein the plurality of processing units (92) controls the electrical power supplied to each of the electronic display (24), the plurality of monitoring devices (28, 32, 34, 36) and the plurality of processing units (92), wherein the plurality of processing units (92) are configured to enter a sleep mode which turns OFF one or more of the plurality of processing units (92), backlighting of the electronic display (24) and the plurality of monitoring devices (28, 32, 34, 36), and wherein the first processing unit (52) remains turned ON in the sleep mode, and when a use mode is requested, the first processing unit (52) turns ON the one or more of the plurality of processing units (92), the backlighting of the electronic display (24) and the plurality of monitoring devices (28, 32, 34, 36).

2. The medical device (20) of claim 1, wherein a processing speed of the first processing unit (52) is reduced in the sleep mode compared to the use mode.

3. The medical device (20) of claim 1 or claim 2, further comprising one or more power ports (46) configured to power one or more electrical loads (90).

4. The medical device (20) of claim 3, wherein the plurality of processor units (92) further turns OFF the power supplied to the one or more power ports (46) in the sleep mode, and reactivates the one or more power ports (46) when the use mode is requested.

5. The medical device (20) of any one of claims 1-4, wherein the plurality of processor units (92) includes the first processing unit (52), the second processing unit (54), a third processing unit and a fourth processing unit, and wherein the second, third and fourth processing units (52, 54, 56, 58) are turned OFF in the sleep mode.

6. The medical device (20) of any one of claims 1-5, wherein the medical device is a medical monitor device (20) having the plurality of monitoring devices (28, 32, 34, 36) configured to monitor medical conditions of a patient.

7. The medical device (20) of claim 6, wherein the plurality of monitoring devices (28, 32, 34, 36) comprises one or more of a temperature sensor monitor (32), a blood pressure sensor monitor (36), an oxygen sensor monitor (34) and a carbon dioxide sensor monitor (28).

8. The medical device (20) of claim 6, wherein the plurality of monitoring devices (28, 32, 34, 36) comprises a temperature sensor monitor (32), a blood pressure sensor monitor (36) and an oxygen sensor monitor (34).

9. The medical device (20) of any one of claims 1-8, further comprising communication circuitry (82) for wireless communication, wherein the communication circuitry (82) remains ON in the sleep mode.

10. The medical device (20) of any one of claims claim 1-9, wherein the battery (80) comprises a plurality of battery cells (80A-80I).

11. The medical device (20) of claim 10, wherein the plurality of battery cells (80A-80I) comprises a plurality of lithium ion battery cells.

12. A method (100) of operating a battery powered medical device (20) having a battery (80), an electronic display (24), a plurality of monitoring devices (28, 32, 34, 36) and a plurality of processing units (92), the method (100) comprising the steps of:
charging the battery (80);
turning ON (104) the electronic display (24), the plurality of monitoring devices (28, 32, 34, 36) and the plurality of processing units (92) in an active use mode;
detecting (108) non-use of the medical device (20) during a first time period or a user request for sleep mode and entering the sleep mode;
turning OFF (116) one or more of the plurality of processing units (92) and maintaining at least a first processing unit (52) turned ON in the sleep mode;
turning OFF (112) backlighting of the electronic display (24) in the sleep mode;
turning OFF (120) the plurality of monitoring devices (28, 32, 34, 36) in the sleep mode;
detecting (122) a request for the active use mode of the medical device (20) while in the sleep mode; and
turning ON (130) all of the plurality of processing units (92), the backlighting of the electronic display (24) and the plurality of monitoring devices (28, 32, 34, 36) in the active use mode.

13. The method (100) of claim 12, further comprising the step of reducing (114) a processing speed of the first processing unit (52) in the sleep mode compared to the active use mode.

14. The method (100) of either claim 12 or claim 13, wherein the plurality of monitoring devices (28, 32, 34, 36) are configured for monitoring medical conditions of a patient.
